(19) Europäisches Patentamt
European Patent Office
Office européen des brevets

(11) EP 0 604 301 B1

(12) # FASCICULE DE BREVET EUROPEEN

(45) Date de publication et mention de la délivrance du brevet: **29.05.1996 Bulletin 1996/22**

(51) Int. Cl.[6]: **C07C 229/42**, A61K 31/23, C07C 271/22, C07C 233/36, A61K 31/215

(21) Numéro de dépôt: **93403109.7**

(22) Date de dépôt: **21.12.1993**

(54) **Nouveaux dérivés de p-[di(2-chloroéthyl)amino]phénylalanine, leur procédé de préparation et les compositions pharmaceutiques qui les contiennent**

p-[Di(2-Chloroethyl)amino]phenylalanin-Derivate, Verfahren zu ihrer Herstellung und diese enthaltende pharmazeutische Zusammensetzungen

p-Di[2-chloroethyl)amino]phenylalanine derivatives process for their preparation and pharmaceutical compositions containing them

(84) Etats contractants désignés:
**AT BE CH DE DK ES FR GB GR IE IT LI LU NL PT SE**

(30) Priorité: **21.12.1992 FR 9215319**

(43) Date de publication de la demande:
**29.06.1994 Bulletin 1994/26**

(73) Titulaire: **ADIR ET COMPAGNIE**
**F-92415 Courbevoie Cédex (FR)**

(72) Inventeurs:
- **Cordi, Alex**
  **F-92150 Suresnes (FR)**
- **Morris, Angela D.**
  **F-78220 Viroflay (FR)**
- **Atassi, Ghanem**
  **F-92400 Saint-Cloud (FR)**

(56) Documents cités:
- **ARZNEIM.-FORSCH., (ARZNAD,00044172), 92, Vol. 42 (9), pages 1153-1156, J. R. DEVERRE et al, "Synthesis of the orally macrofilaricidal and stable glycerolipidic prodrug of melphalan, 1,3-dipalmitoyl-2-(4'-(bis(2"-chloroethyl)amino)]phenylalaninoyl)glycerol"**
- **JOURNAL OF MEDICINAL CHEMISTRY, vol. 26, no. 8, 1983, Washington, US, pages 1200-1203, A. GARZON-ABURBEH et al, "1,3-Dipalmitoylglycerol ester of chlorambucil as a lymphotropic, orally administrable antineoplastic agent"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 604 301 B1

Printed by Rank Xerox (UK) Business Services
2.11.7/3.4

## Description

La présente invention concerne de nouveaux dérivés de p-[bis(2-chloroéthyl)amino]phénylalanine, leur procédé de préparation et les compositions pharmaceutiques qui les contiennent.

Un certain nombre de prodrogues glycérolipidiques ont été déjà décrites dans la littérature dans des domaines aussi variés que ceux des antiinflammatoires non stéroidiens, des bactéricides ou bien encore des anticancéreux. Ces prodrogues ont pour but de diminuer, voire de supprimer, certains effets secondaires des principes actifs à partir desquels elles sont synthétisées.

Les prodrogues glycérolipidiques ont pour objectif de cibler le système lymphatique à partir de la voie orale, d'éviter les effets de premier passage hépatique ainsi que d'améliorer la biodisponibilité orale de certains médicaments.

Dans le domaine des anticancéreux, il était intéressant de synthétiser des prodrogues capables d'atteindre des tumeurs à dissémination lymphatique et/ou de franchir la barrière hématoencéphalique après administration par voie orale. C'est le cas, par exemple, des esters du chlorambucil décrits par A. Garzon-Aburdeh (J. Med. Chem., 26, 1200-1203, 1983).

Des prodrogues du Melphalan ont, quant à elles, été décrites pour une utilisation dans le domaine antiparasitaire (J.R. DEVERRE, Arzneim.-Forsch/Drug Res. 42(II), 9, 1153-1156, 1992).

Les composés de la présente invention sont des prodrogues du Melphalan qui, outre le fait qu'elles soient nouvelles, présentent une activité pharmacologique particulièrement intéressante.

Plus spécifiquement, la présente invention concerne les composés de formule (I) :

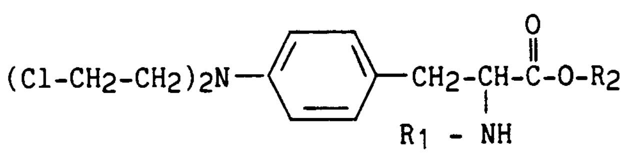

(I)

dans laquelle :

$R_1$ représente un atome d'hydrogène, un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy ou trihalogénoalkyle),

$R_2$ représente l'un quelconque des groupements suivants :

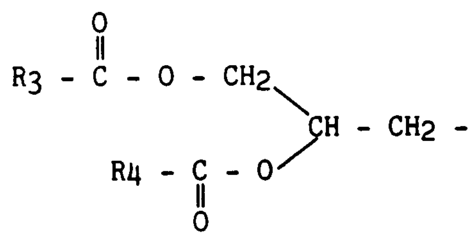

(A)

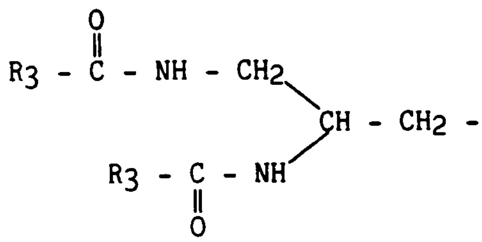

(B)

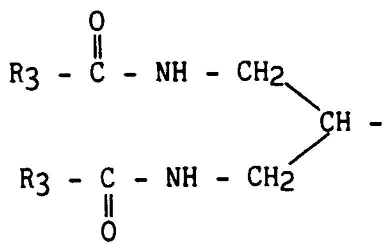

(C)

dans lesquels :

$R_3$ ou $R_4$, identiques ou différents, représentent un radical alkyle ($C_6$-$C_{19}$) linéaire ou ramifié ou alkényle ($C_6$-$C_{19}$) linéaire ou ramifié,

leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

L'invention s'étend également au procédé de préparation des composés de formule (I).

Le procédé de préparation des composés de formule (I) dans laquelle $R_2$ représente le groupement (A) est caractérisé en ce que l'on utilise comme produit de départ la p-[bis(2-chloroéthyl)amino]phénylalanine de formule (II), sous forme racémique ou d'énantiomère pur, dont on a protégé la fonction amine par un groupement protecteur classique :

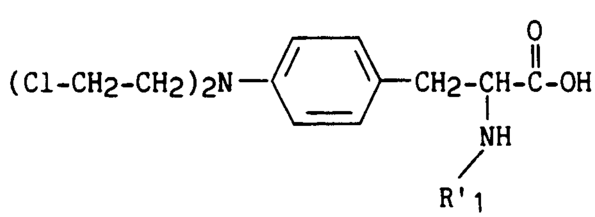

(II)

dans laquelle $R'_1$ représente un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle), que l'on fait réagir sur le 1,3-dioxolane de formule (III) sous forme racémique ou d'énantiomère pur :

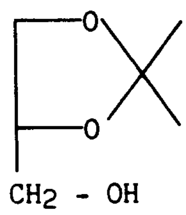

(III)

en présence d'un agent de couplage de la synthèse peptidique comme le benzotriazol-1-yloxy-trisdiméthylaminophosphoniumhexafluorophosphate (BOP) (selon la technique décrite dans Tet. Lett. 1219, 1975) et de diisopropyléthylamine dans du dichlorométhane,
pour conduire au 1,3-dioxolane de formule (IV) :

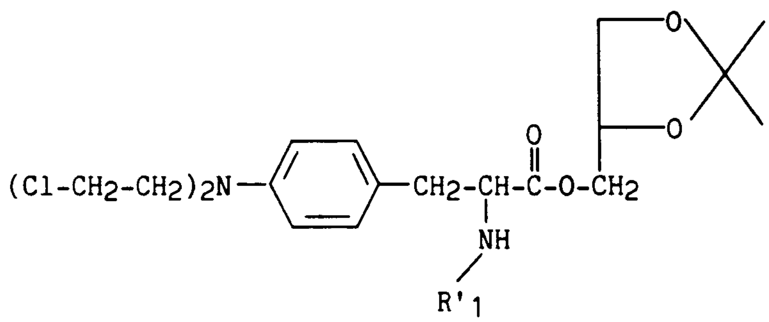

(IV)

dans laquelle $R'_1$ a la même signification que précédemment,
que l'on transforme, en milieu acide, en diol correspondant de formule (V) :

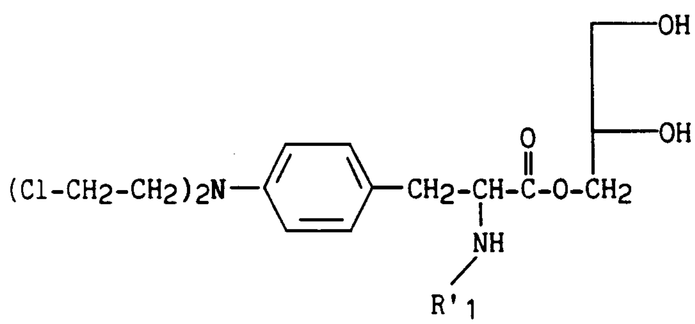

(V)

dans laquelle $R'_1$ a la même signification que précédemment,
que l'on fait réagir selon la nature des composés de formule (I) que l'on souhaite obtenir avec :

- soit, dans le cas où $R_3$ et $R_4$ sont identiques :
  2 équivalents du chlorure d'acide de formule (VI), en présence d'une base,

$$R_3 - CO - Cl \qquad \text{(VI)}$$

  dans laquelle $R_1$ a la même signification que dans la formule (I),
  pour conduire au composé de formule (I/a), cas particulier des composés de formule (I),

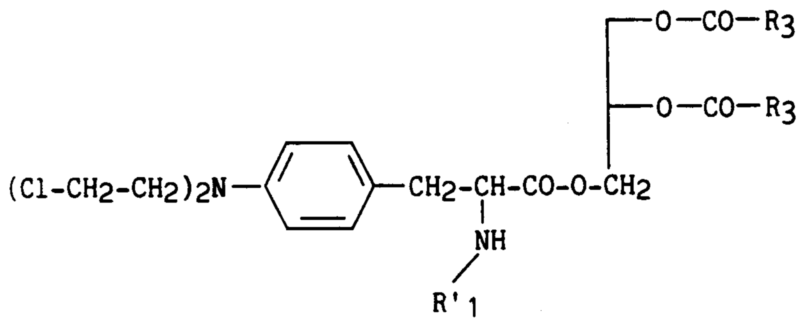

(I/a)

dans laquelle $R'_1$ et $R_3$ ont la même signification que précédemment,

- soit, dans le cas où $R_3$ et $R_4$ sont différents :
  avec un équivalent du chlorure d'acide de formule (VI) décrit précédemment,
  pour conduire, après séparation des composés mono et diacylés, au composé de formule :

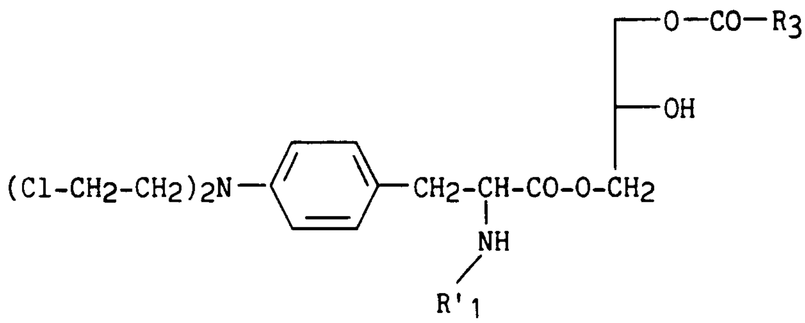

(VII)

dans laquelle $R'_1$ et $R_3$ ont la même signification que précédemment,
que l'on fait réagir avec un équivalent du chlorure d'acide de formule (VIII) en présence d'une base :

$$R_4 - CO - Cl \qquad \text{(VIII)}$$

pour conduire au composé de formule (I/b), cas particulier des composés de formule (I),

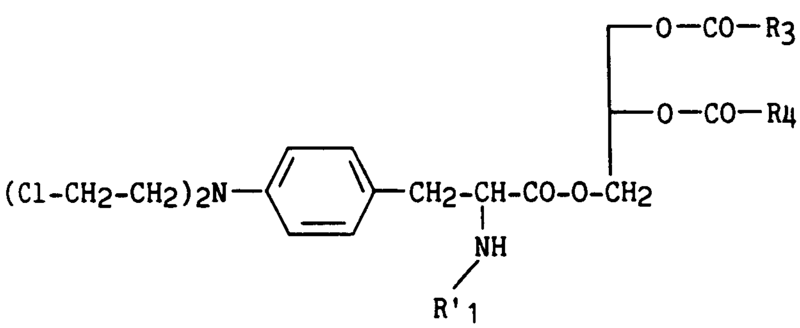

(I/b)

dans laquelle $R'_1$, $R_3$ et $R_4$ ont la même signification que précédemment,

composés de formule (I/a) ou (I/b), qui peuvent subir, si on le souhaite, un hydrolyse acide ou une hydrogénolyse pour conduire respectivement aux composés de formule (I/c) ou (I/d), cas particulier des composés de formule (I) :

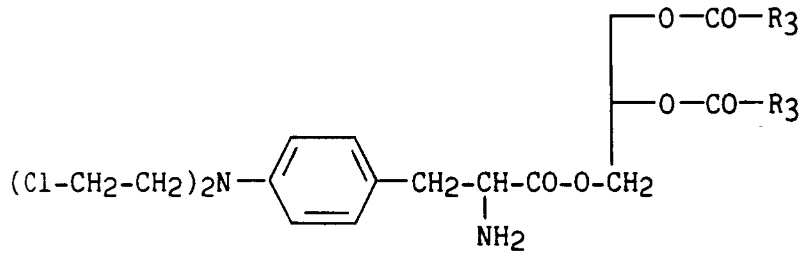

(I/c)

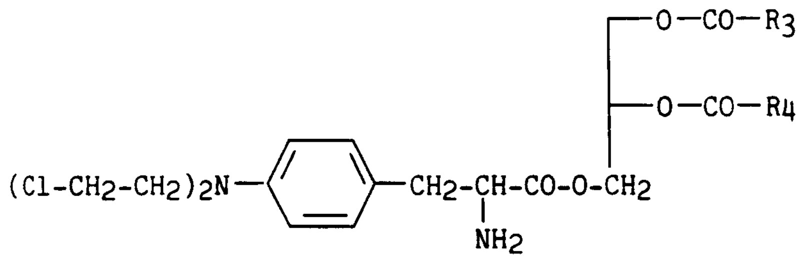

(I/d)

dans lesquelles $R_3$ et $R_4$ ont la même signification que dans la formule (I), composés de formule (I/a), (I/b), (I/c) ou (I/d) :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

Un autre procédé de synthèse des composés de formule (I) dans laquelle $R_2$ représente le groupement (A) est caractérisé en ce que l'on utilise comme produit de départ un composé de formule (IX), sous forme racémique ou d'énantiomère pur, préparé selon l'un ou l'autre des procédés décrits par J. GIGG et coll. (J.C.S., 431-434, 1967) ou F.R. PFEIFFER et coll. (Tett. Let., 32, 3549-3552, 1968) :

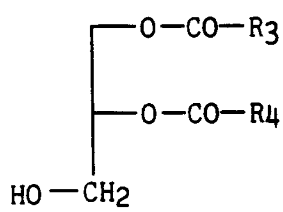

(IX)

dans laquelle $R_3$ et $R_4$ ont la même signification que dans la formule (I), que l'on fait réagir, en présence d'un agent de couplage de la synthèse peptidique comme le benzotriazol-1-yloxy-trisdiméthylaminophosphonium-mhexafluorophosphate (BOP) (selon la technique décrite dans Tet. Lett. 1219, 1975) et de diisopropyléthylamine dans du dichlorométhane, sur la p-[bis(2-chloroéthyl)amino]phénylalanine de formule (II), sous forme racémique ou d'énantiomère pur, dont on a protégé la fonction amine par un groupement protecteur classique :

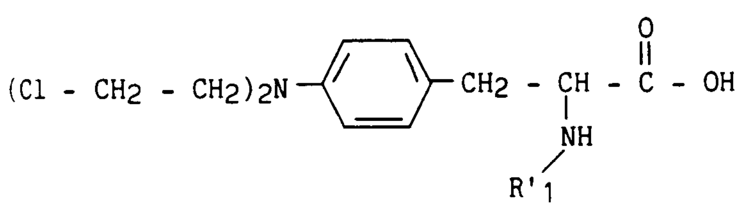

(II)

dans laquelle R'$_1$ représente un radical alkoxy carbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle), pour conduire au composé de formule (I'), cas particulier des composés de formule (I) :

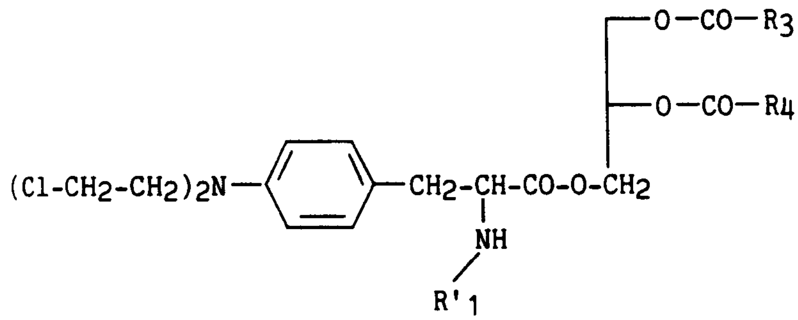

(I')

dans laquelle R'$_1$, $R_3$, $R_4$ ont la même signification que précédemment,
qui peut subir, si on le souhaite, une hydrolyse acide ou une hydrogénolyse, pour conduire respectivement au composé de formule (I"), cas particulier des composés de formule (I) :

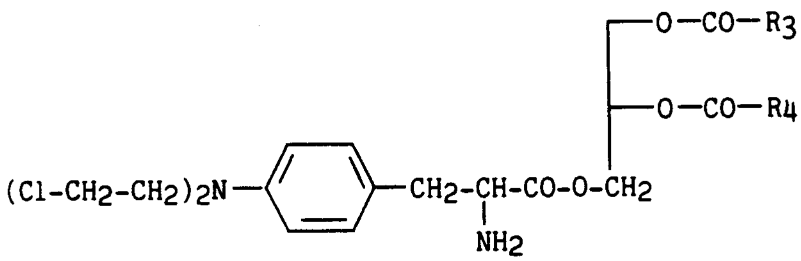

(I")

dans laquelle $R_3$ et $R_4$ ont la même signification que dans la formule (I),
composés de formule (I') ou (I") :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

Le procédé de préparation des composés de formule (I) dans laquelle $R_2$ représente le groupement (B) ou (C) est caractérisé en ce que l'on utilise comme produit de départ la p-[bis(2-chloroéthyl)amino]phénylalanine de formule (II), sous forme racémique ou d'énantiomère pur, dont on a protégé la fonction amine par un groupement protecteur classique :

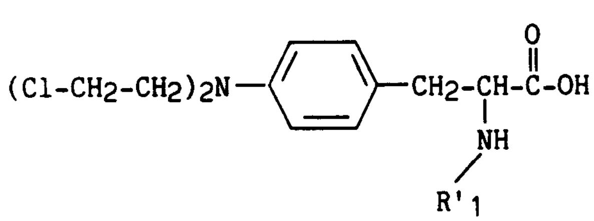

(II)

dans laquelle R'$_1$ représente un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle), que l'on fait réagir en présence de chloroformate d'isoprényl, de triéthylamine et de 4-diméthylaminopyridine :

- soit, sur le composé de formule (Xa) :

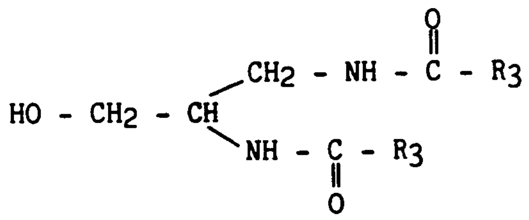

(Xa)

- soit, sur le composé de formule (Xb) :

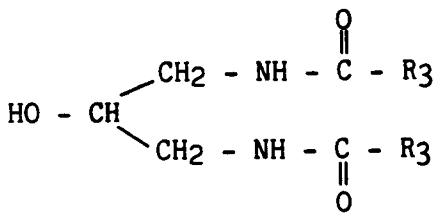

(Xb)

pour conduire respectivement aux composés de formule (I/e) ou (I/f), cas particulier des composés de formule (I) :

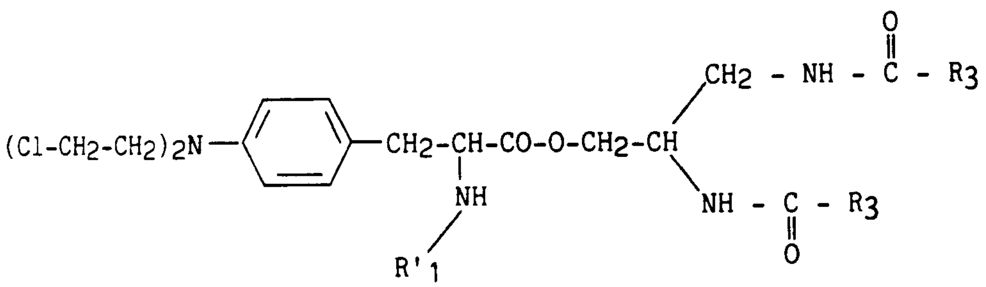

(I/e)

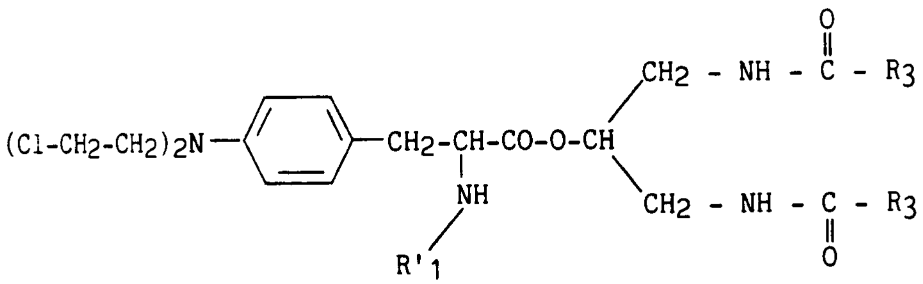

(I/f)

composés de formule (I/e) ou (I/f), qui peuvent subir, si on le souhaite, une hydrolyse acide ou une hydrogénolyse, pour conduire respectivement aux composés de formule (I/g) ou (I/h), cas particulier des composés de formule (I) :

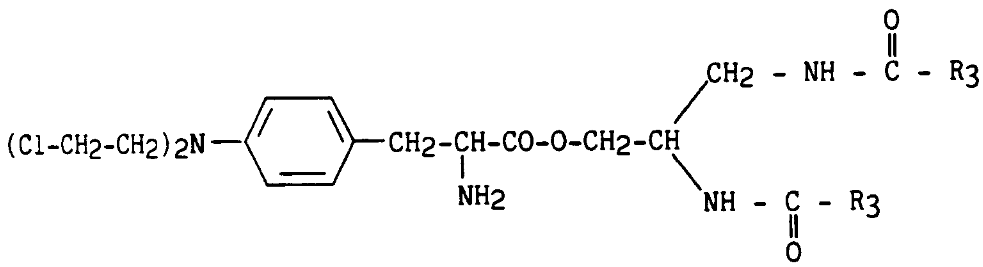

(I/g)

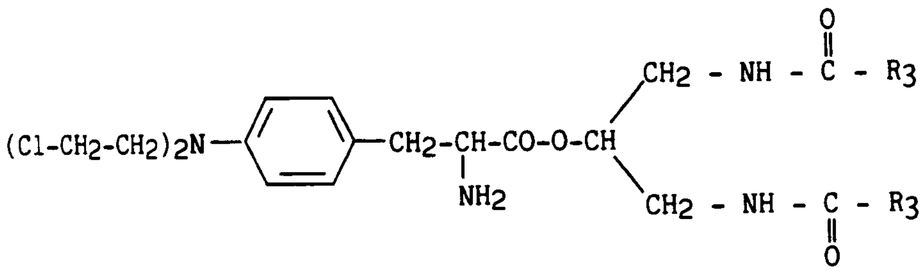

(I/h)

composés de formule (I/e), (I/f), (I/g) ou (I/h) :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

Ces nouveaux dérivés de p-[bis(2-chloroéthyl)amino]phénylalanine présentent des propriétés pharmacologiques très intéressantes. Ils présentent d'une part une activité très supérieure à celle du principe actif à partir duquel ils sont synthétisés, et d'autre part une toxicité nettement moins élevée.

L'invention s'étend aussi aux compositions pharmaceutiques renfermant comme principe actif au moins un composé de formule (I) ou ses isomères optiques avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, etc...

La posologie utile est adaptable selon la nature et la sévérité de l'affection, la voie d'administration ainsi que selon l'âge et le poids du patient. Cette posologie unitaire varie de 10 mg à 1 g par jour en une ou plusieurs prises.

Les exemples suivants illustrent l'invention et ne la limitent en aucune façon.

Les produits de départ utilisés sont des produits connus ou préparés selon des modes opératoires connus.

**EXEMPLE 1 : 1,2-Distéaroyl-3-{(S)-N-*tert*-butoxycarbonyl-4-[bis(2-chloroéthyl)amino]phénylalanyl}glycérol**

Stade A : (S)-N-*tert*-Butoxycarbonyl-4-[bis(2-chloroéthyl)amino] phénylalanine

A une solution maintenue à 0°C, sous agitation, contenant 10 mmoles de (S)-4-[bis(2-chloroéthyl)amino]phénylalanyle dans 20 ml de dioxane, 10 ml d'eau et 11 ml de NaOH 1N, sont additionnées 11 mmoles di-tert-butyldicarbonate. Après 16 heures d'agitation à 20°C, l'ensemble est concentré et le résidu est repris dans 12 ml d'eau et 75 ml d'acétate d'éthyle. Le pH est ramené à 2 et la phase aqueuse extraite avec 3 x 200 ml d'acétate d'éthyle. La phase organique est lavée par 200 ml d'eau et 200 ml d'une solution saturée de chlorure de sodium, séchée puis concentrée sous vide.

Stade B : 1,2-Distéaroyl-3-{(S)-N-*tert*-butoxycarbonyl-4-[bis(2-chloroéthyl)amino]phénylalanyl}glycérol

8 mmoles du composé obtenu au stade précédent, 8 mmoles de benzotriazol-1-yloxy-tris(diméthylamino)phosphonium hexafluorophosphate et 8 mmoles de 1,2-distéaroylglycérol sont mélangées dans 50 ml de dichlorométhane. 16 mmoles de diisopropyléthylamine sont ajoutées et la solution est agitée à 20°C pendant 16 heures. Le mélange est dilué avec 150 ml de dichlorométhane puis lavé par 3 x 80 ml d'une solution saturée de chlorure de sodium. La phase organique est séchée puis concentrée sous vide. Le produit est obtenu, sous forme solide, après purification du résidu par chromatographie sur gel de silice en utilisant le dichlorométhane comme éluant.
Point de fusion : 51-52°C

| Microanalyse élémentaire : | | | | |
|---|---|---|---|---|
| | C % | H % | N % | Cl % |
| calculé | 67,63 | 9,96 | 2,77 | 7,00 |
| trouvé | 67,59 | 9,67 | 3,07 | 7,30 |

**EXEMPLE 2 : 1,2-Distéaroyl-3-{(S)-4-[bis(2-chloroéthyl)amino] phénylalanyl}glycérol**

Une solution de 5 mmoles du produit obtenu dans l'exemple 1 dans 25 ml de dioxane saturé en chlorure d'hydrogène est agitée pendant 20 minutes à 20°C. L'ensemble est concentré sous vide et le résidu est lavé avec du pentane, pour conduire au produit attendu sous forme de chlorhydrate.
Point de fusion : 82-83°C

| Microanalyse élémentaire : | | | | |
|---|---|---|---|---|
| | C % | H % | N % | Cl % |
| calculé | 65,84 | 9,88 | 2,95 | 11,21 |
| trouvé | 65,50 | 9,70 | 3,08 | 11,27 |

Les composés des exemples 3 et 4 ont été obtenu selon le même procédé que celui de l'exemple 1 en utilisant les produits de départ correspondant.

**EXEMPLE 3 : 1,2-Dipalmitoyl-3-{(S)-N-*tert*-butoxycarbonyl-4-[bis(2-chloroéthyl)amino]phénylalanyl}glycérol**

Point de fusion : 36-37°C

| Microanalyse élémentaire : | | | | |
|---|---|---|---|---|
| | C % | H % | N % | Cl % |
| calculé | 66,57 | 9,70 | 2,93 | 7,42 |
| trouvé | 66,44 | 9,32 | 3,27 | 7,58 |

**EXEMPLE 4 : 1,2-Dimyristoyl-3-{(S)-N-*tert*-butoxycarbonyl-4-[bis(2-chloroéthyl)amino]phénylalanyl}glycérol**

Point de fusion : huile

| Microanalyse élémentaire : | | | | |
|---|---|---|---|---|
| | C % | H % | N % | Cl % |
| calculé | 65,38 | 9,41 | 3,11 | 7,88 |
| trouvé | 65,38 | 9,32 | 3,47 | 8,21 |

**EXEMPLE 5 : 1,2-Dipalmitoyl-3-{(S)-4-[bis(2-chloroéthyl)amino] phénylalanyl}glycérol**

Ce composé a été obtenu selon le procédé décrit dans l'exemple 2 à partir du composé de l'exemple 3.
Point de fusion : 85-86°C

| Microanalyse élémentaire : | | | | |
|---|---|---|---|---|
| | C % | H % | N % | Cl % |
| calculé | 64,59 | 9,60 | 3,14 | 11,92 |
| trouvé | 64,21 | 9,66 | 3,15 | 11,67 |

**EXEMPLE 6 : 1,2-Dimyristoyl-3-{(S)-4-[bis(2-chloroéthyl)amino] phénylalanyl}glycérol**

Ce composé a été obtenu selon le procédé décrit dans l'exemple 2 à partir du composé de l'exemple 4.
Point de fusion : 91-92°C

| Microanalyse élémentaire : | | | | |
|---|---|---|---|---|
| | C % | H % | N % | Cl % |
| calculé | 63,18 | 9,28 | 3,35 | 12,72 |
| trouvé | 62,65 | 9,00 | 3,31 | 12,59 |

**EXEMPLE 7 : 2,3-Bis(palmitoylamino)-(O)-{(S)-N-*tert*-butoxycarbonyl-4-[bis(2-chloroéthyl)amino]phénylalanyl}propan-1-ol**

8 mmoles de (S)-N-*tert*-butoxycarbonyl-4-[bis(2-chloroéthyl)amino]phénylalanine, 8,8 mmoles de 2,3-bis(palmitoylamino)propan-1-ol, 8,8 mmoles de triéthylamine, 4,4 mmoles de 4-diméthylaminopyridine sont mélangées dans 50 ml de tétrahydrofurane à 35-40°C pendant 20 minutes. 8,8 mmoles de chloroformate d' isoprényl sont ajoutées et la solution est agitée 90 minutes. L'ensemble est alors concentré et le résidu est repris dans 100 ml de dichlorométhane, lavé avec 100 ml d'eau puis par une solution de bicarbonate de sodium à 10 % et enfin par une solution saturée de chlorure de sodium. La phase organique est séchée puis concentrée sous vide. Le produit attendu est alors obtenu, sous forme solide, après purification du résidu par chromatographie sur gel de silice en utilisant comme éluant un mélange dichlorométhane/éthanol (95/5).
Point de fusion : 79-80°C

**EXEMPLE 8 : 2,3-Bis(palmitoylamino)-(O)-{(S)-4-[bis(2-chloroéthyl)amino] phénylalanyl}propan-1-ol, chlorhydrate**

Une solution contenant 5 mmoles du produit décrit dans l'exemple 7 dans 25 ml de dioxane saturé par de l'acide chlorhydrique gazeux est agitée 20 minutes à 20°C. Après concentration du solvant, on obtient le produit attendu sous forme de chlorhydrate.
Point de fusion : 130-132°C

| Microanalyse élémentaire : | | | |
|---|---|---|---|
| | C % | H % | N % |
| calculé | 64,73 | 9,85 | 6,29 |
| trouvé | 64,42 | 9,56 | 6,00 |

**EXEMPLE 9 : 1,3-Bis(palmitoylamino)-(O)-{(S)-N-*tert*-butoxycarbonyl-4-[bis(2-chloroéthyl)amino]phénylalanyl}isopropanol**

Le produit attendu est obtenu selon le procédé décrit dans l'exemple 7 en remplaçant le 2,3-bis(palmitoylamino)propan-1-ol par le 1,3-bis(palmitoyl-amino)isopropanol.
Point de fusion : 88-89°C

**EXEMPLE 10 : 1,3-Bis(palmitoylamino)-(O)-{(S)-4-[bis(2-chloroéthyl)amino] phénylalanyl}isopropanol**

Le produit attendu est obtenu selon le procédé décrit dans l'exemple 8 en utilisant le composé de l'exemple 9.
Point de fusion : 153-155°C

| Microanalyse élémentaire : | | | | |
|---|---|---|---|---|
| | C % | H % | N % | Cl % |
| calculé | 64,73 | 9,85 | 6,29 | 11,94 |
| trouvé | 64,57 | 9,82 | 6,10 | 12,11 |

**ETUDE PHARMACOLOGIQUE DES COMPOSES DE L'INVENTION**

**EXEMPLE 11 : Evaluation de l'activité antitumorale sur la leucémie P388 (i.p)**

L'étude a été réalisée selon le protocole suivant :

$10^6$ cellules leucémiques (P388) sont inoculées au jour 0 par voie intrapéritonéale (i.p) à des souris BDFa femelles de poids moyen compris entre 18 et 22 g.

Les produits sont administrés, au jour 1, par voie i.p, en dose unique.

Les critères d'activité sont le nombre de survivants à long terme dans les groupes traités et le rapport T/C % :

$$\frac{T}{C}(\%) = \frac{\text{survie médiane des souris traitées}}{\text{survie médiane des souris contrôles}}$$

Le rapport T/C doit être supérieur ou égal à 125 % et un rapport T/C inférieur ou égal à 95 % indique la présence d'une toxicité générale comme l'ont montré GERAN R.I. et coll. (Cancer Chemoter. Rep. 3 (3), 1-100, 1972).

Les résultats rassemblés ci-dessous montrent que ces prodrogues présentent une activité particulièrement puissante associée à une toxicité nettement moins élevée que celle du melphalan.

| Exemple | Dose (mg/kg) | Var. Poids (g) J1-J5 | T/C méd. (%) | Survie à J35 |
|---|---|---|---|---|
| 2 | 46,6 | + 0,5 | 199 | 0/5 |
| | 93,3 | - 1,0 | 273 | 1/5 |
| 5 | 43,9 | + 0,2 | 245 | 2/5 |
| | 87,8 | - 0,5 | > 330 | 5/5 |
| 6 | 20,4 | + 0,7 | > 330 | 6/8 |
| | 40,8 | + 0,4 | > 330 | 8/8 |
| | 81,7 | - 2,7 | > 330 | 8/8 |
| Melphalan | 7,5 | + 0,6 | 231 | 3/8 |
| | 15 | + 0,1 | > 330 | 7/8 |
| | 30 | - 4,2 | 93 | 2/8 |
| Témoins | - | + 2,6 | 100 | 0/24 |

(Les doses indiquées dans ce tableau sont calculées en EQUIVALENTS Melphalan).

**EXEMPLE 12** : **Composition pharmaceutique**

Formule de préparation pour 1000 comprimés dosés 100 mg :

| | |
|---|---|
| Composé de l'exemple 2 | 100 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I) :

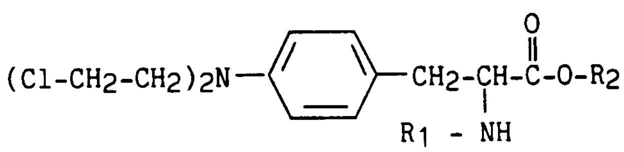

(I)

dans laquelle :

$R_1$ représente un atome d'hydrogène, un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy ou trihalogénoalkyle),

$R_2$ représente l'un quelconque des groupements suivants :

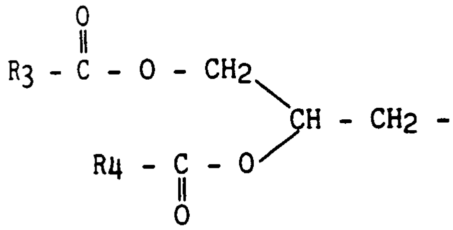

(A)

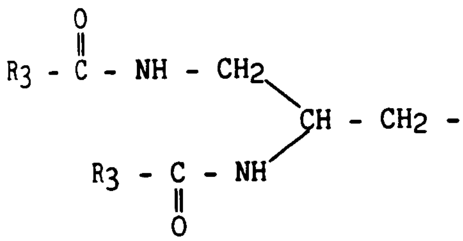

(B)

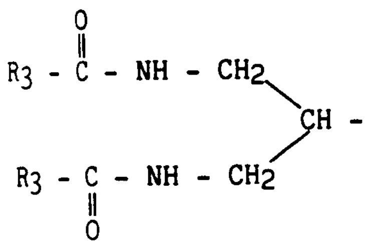

(C)

dans lesquels :

$R_3$ ou $R_4$, identiques ou différents, représentent un radical alkyle ($C_6$-$C_{19}$) linéaire ou ramifié ou alkényle ($C_6$-$C_{19}$) linéaire ou ramifié,

leurs énantiomères, diastéréoisomères et épimères ainsi que leurs sels d'addition à un acide pharmaceutiquement acceptable.

**2.** Composé de formule (I) selon la revendication 1 dans laquelle $R_2$ représente le groupement

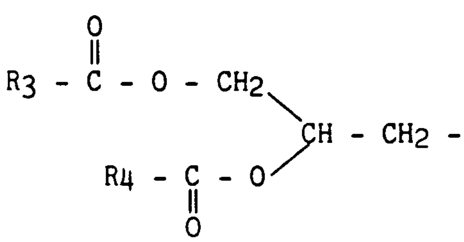

,

ses énantiomères et diastéréoisomères ainsi que ses sels d'addition à un acide pharmaceutiquement acceptable.

**3.** Composé de formule (I) selon la revendication 1 dans laquelle $R_3$-CO et $R_4$-CO, identiques, représentent chacun un groupement stéaroyl, ses énantiomères et diastéréoisomères ainsi que ses sels d'addition à un acide pharmaceutiquement acceptable.

**4.** Composé de formule (I) selon la revendication 1 dans laquelle $R_3$-CO et $R_4$-CO, identiques, représentent chacun un groupement myristoyl, ses énantiomères et diastéréoisomères ainsi que ses sels d'addition à un acide pharmaceutiquement acceptable.

**5.** Composé de formule (I) selon la revendication 1 dans laquelle $R_3$-CO et $R_4$-CO, identiques, représentent chacun un groupement palmitoyl, ses énantiomères et diastéréoisomères ainsi que ses sels d'addition à un acide pharmaceutiquement acceptable.

**6.** Composés de formule (I) selon la revendication 1 dans laquelle $R_1$ représente un atome d'hydrogène, leurs énantiomères, diastéréoisomères ainsi que leurs sels d'addition à un acide pharmaceutiquement acceptable.

**7.** Procédé de préparation des composés de formule (I) selon la revendication 1 dans laquelle $R_2$ représente le groupement (A), caractérisé en ce que l'on utilise comme produit de départ la p-[bis(2-chloroéthyl)amino]phénylalanine de formule (II), sous forme racémique ou d'énantiomère pur, dont on a protégé la fonction amine par un groupement protecteur classique :

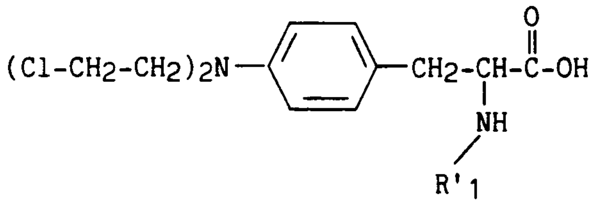

(II)

dans laquelle $R'_1$ représente un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle),
que l'on fait réagir sur le 1,3-dioxolane de formule (III) sous forme racémique ou d'énantiomère pur :

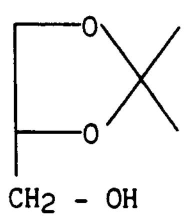

(III)

en présence d'un agent de couplage de la synthèse peptidique comme le benzotriazol-1-yloxy-trisdiméthylaminophosphoniumhexafluorophosphate (BOP) et de diisopropyléthylamine dans du dichlorométhane,
pour conduire au 1,3-dioxolane de formule (IV) :

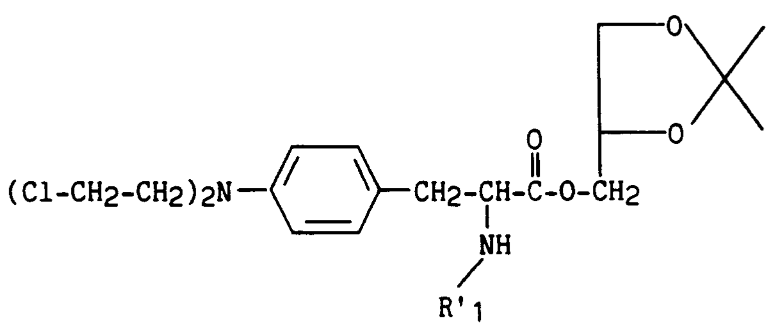

(IV)

dans laquelle $R'_1$ a la même signification que précédemment,
que l'on transforme, en milieu acide, en diol correspondant de formule (V) :

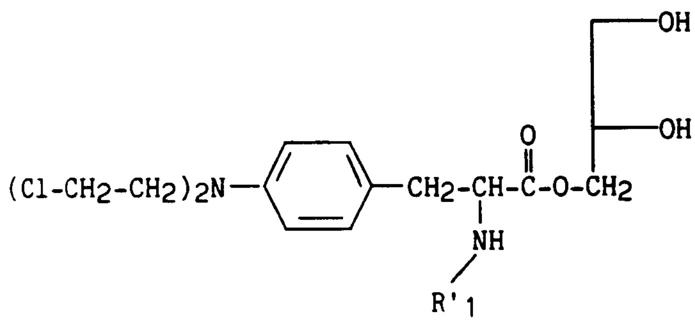

(V)

dans laquelle $R'_1$ a la même signification que précédemment,
que l'on fait réagir selon la nature des composés de formule (I) que l'on souhaite obtenir avec :

- soit, dans le cas où $R_3$ et $R_4$ sont identiques :
  2 équivalents du chlorure d'acide de formule (VI), en présence d'une base,

  $$R_3 - CO - Cl \qquad (VI)$$

  dans laquelle $R_1$ a la même signification que dans la formule (I),
  pour conduire au composé de formule (I/a), cas particulier des composés de formule (I),

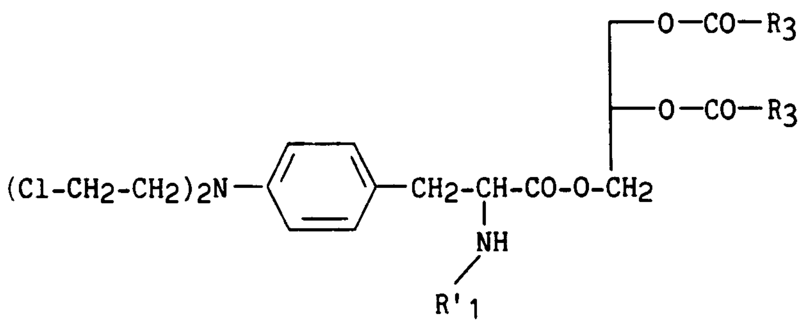

(I/a)

  dans laquelle $R'_1$ et $R_3$ ont la même signification que précédemment,
- soit, dans le cas où $R_3$ et $R_4$ sont différents :
  avec un équivalent du chlorure d'acide de formule (VI) décrit précédemment,
  pour conduire, après séparation des composés mono et diacylés, au composé de formule :

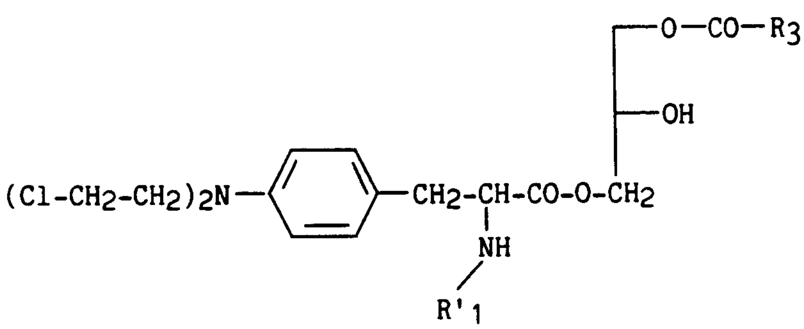

(VII)

  dans laquelle $R'_1$ et $R_3$ ont la même signification que précédemment,
  que l'on fait réagir avec un équivalent du chlorure d'acide de formule (VIII) en présence d'une base :

  $$R_4 - CO - Cl \qquad (VIII)$$

pour conduire au composé de formule (I/b), cas particulier des composés de formule (I),

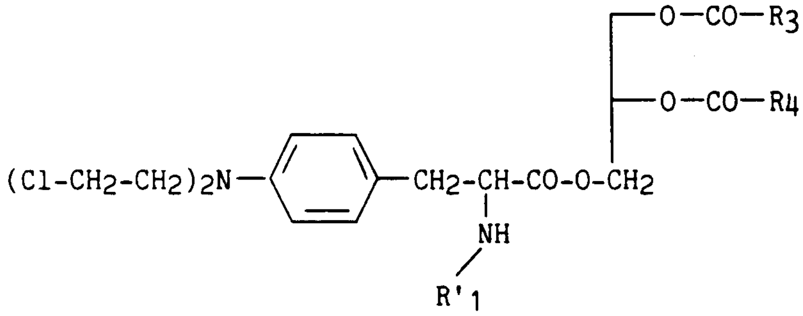

(I/b)

dans laquelle $R'_1$, $R_3$ et $R_4$ ont la même signification que précédemment,

composés de formule (I/a) ou (I/b), qui peuvent subir, si on le souhaite, un hydrolyse acide ou une hydrogénolyse, pour conduire respectivement aux composés de formule (I/c) ou (I/d), cas particulier des composés de formule (I) :

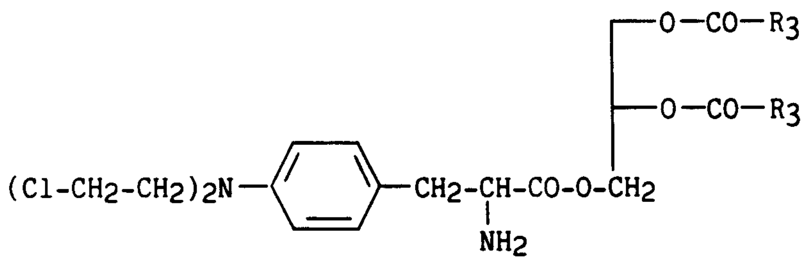

(I/c)

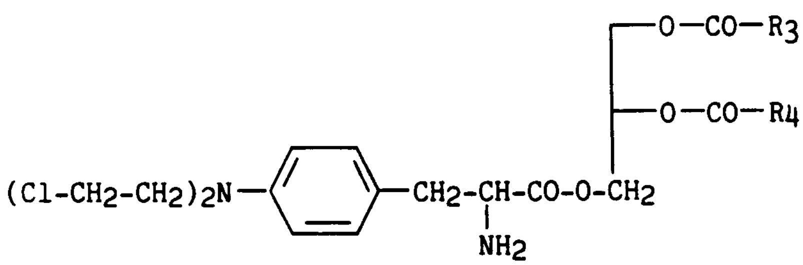

(I/d)

dans lesquelles $R_3$ et $R_4$ ont la même signification que dans la formule (I),
composés de formule (I/a), (I/b), (I/c) ou (I/d) :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

**8.** Procédé de préparation des composés de formule (I) selon la revendication 1 dans laquelle $R_2$ représente le groupement (A), caractérisé en ce que l'on utilise comme produit de départ un composé de formule (IX) :

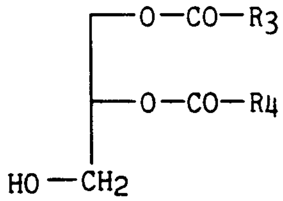

(IX)

dans laquelle $R_3$ et $R_4$ ont la même signification que dans la formule (I), que l'on fait réagir, en présence d'un agent de couplage de la synthèse peptidique comme le benzotriazol-1-yloxy-trisdiméthylaminophosphoniumhexafluorophosphate (BOP) et de diisopropyléthylamine dans du dichlorométhane, sur la p-[bis(2-chloroéthyl)amino]phénylalanine de formule (II), sous forme racémique ou d'énantiomère pur, dont on a protégé la fonction amine par un groupement protecteur classique :

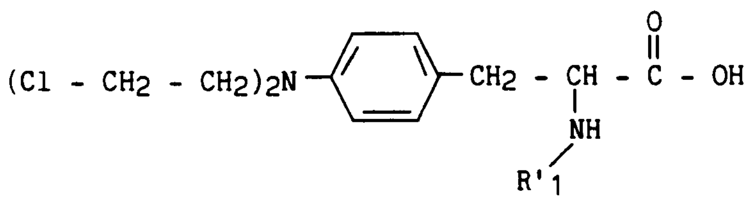

(II)

dans laquelle $R'_1$ représente un radical alkoxy ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle), pour conduire au composé de formule (I'), cas particulier des composés de formule (I) :

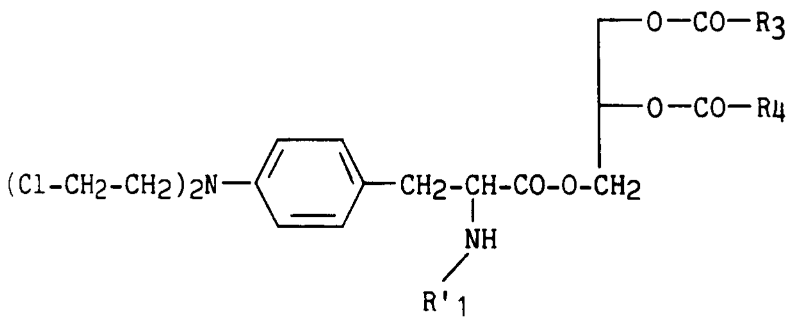

(I')

dans laquelle $R'_1$, $R_3$, $R_4$ ont la même signification que précédemment,
qui peut subir, si on le souhaite, une hydrolyse acide, ou une hydrogénolyse, pour conduire respectivement aux composés de formule (I"), cas particulier des composés de formule (I) :

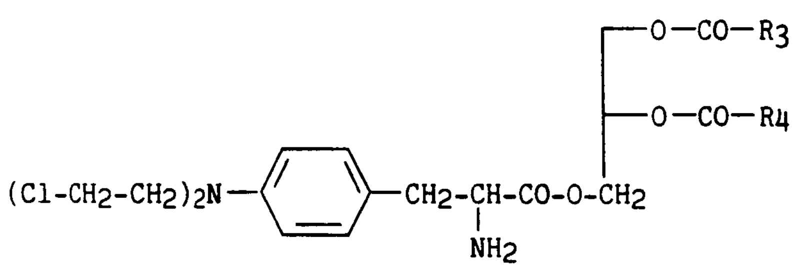

(I")

dans laquelle $R_3$ et $R_4$ ont la même signification que dans la formule (I), composés de formule (I') ou (I'') :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

**9.** Procédé de préparation des composés de formule (I) selon la revendication 1 dans laquelle $R_2$ représente le groupement (B) ou (C) est caractérisé en ce que l'on utilise comme produit de départ la p-[bis(2-chloroéthyl)amino]phénylalanine de formule (II), sous forme racémique ou d'énantiomère pur, dont on a protégé la fonction amine par un groupement protecteur classique :

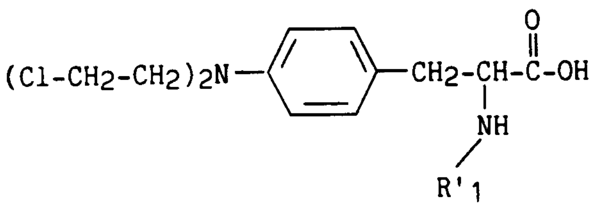

(II)

dans laquelle $R'_1$ représente un radical alkoxycarbonyl ($C_1$-$C_6$) linéaire ou ramifié, ou phénylalkoxycarbonyl (substitué ou non sur le noyau phényl par un ou plusieurs atomes d'halogène ou groupements alkyle, alkoxy, trihalogénoalkyle),
que l'on fait réagir en présence de chloroformate d'isoprényl, de triéthylamine et de 4-diméthylaminopyridine :

- <u>soit</u>, sur le composé de formule (Xa) :

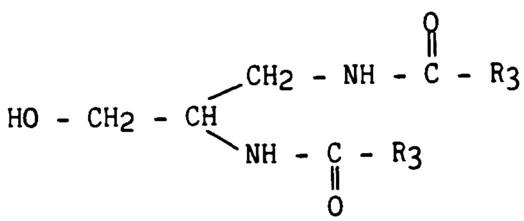

(Xa)

- <u>soit</u>, sur le composé de formule (Xb) :

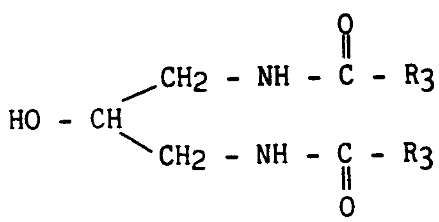

(Xb)

pour conduire respectivement aux composés de formule (I/e) ou (I/f), cas particulier des composés de formule (I) :

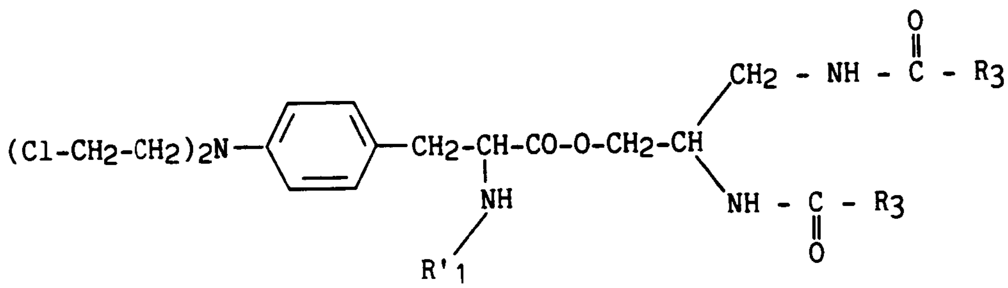

(I/e)

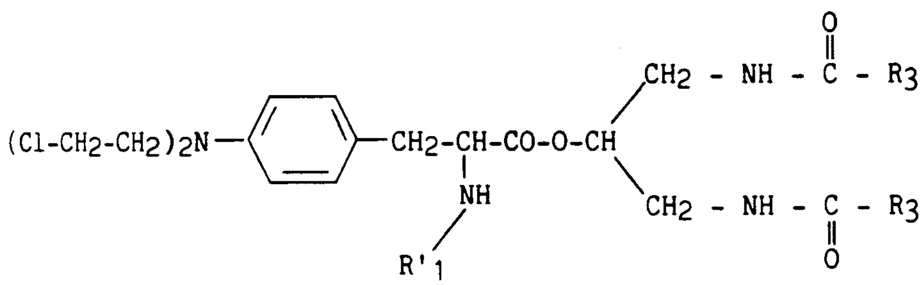

(I/f)

composés de formule (I/e) ou (I/f), qui peuvent subir, si on le souhaite, une hydrolyse acide ou une hydrogénolyse, pour conduire respectivement aux composés de formule (I/g) ou (I/h), cas particulier des composés de formule (I) :

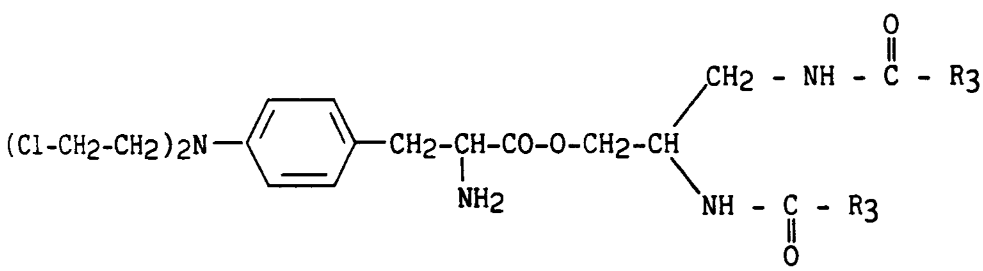

(I/g)

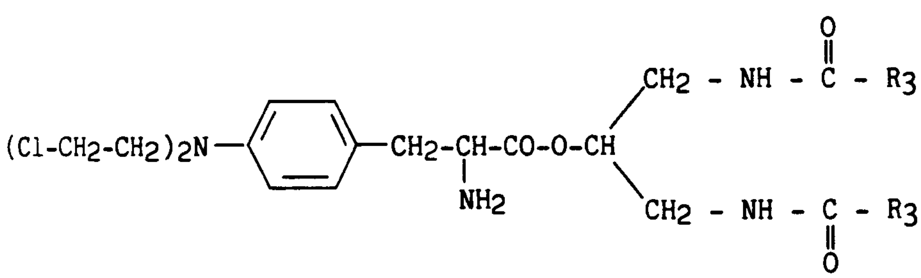

(I/h)

composés de formule (I/e), (I/f), (I/g) ou (I/h) :

- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

**10.** Compositions pharmaceutiques contenant comme principe actif au moins un composé selon l'une quelconques revendications 1 à 6, seul ou en combinaison avec un ou plusieurs véhicules inertes, non toxiques pharmaceutiquement acceptables.

**11.** Compositions pharmaceutiques selon la revendication 10 utiles dans le traitement du cancer.

## Claims

**1.** Compounds of formula I :

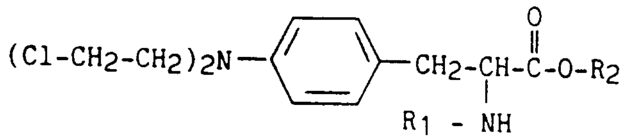

(I)

wherein :

$R_1$ represents a hydrogen atom, a straight-chained or branched ($C_1$-$C_6$)alkoxycarbonyl radical, or a phenylalkoxycarbonyl radical (unsubstituted or substituted on the phenyl nucleus by one or more halogen atoms or alkyl, alkoxy or trihaloalkyl groups),

$R_2$ represents any one of the following groups :

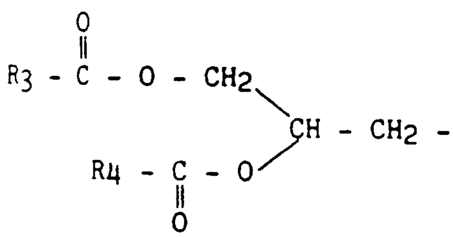

(A)

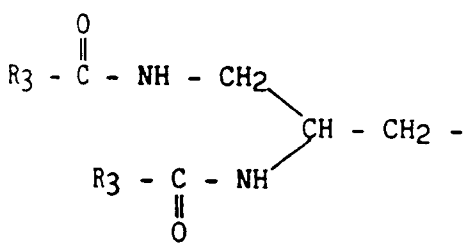

(B)

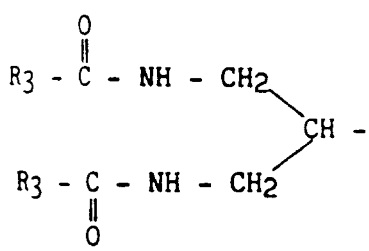

(C)

wherein :

$R_3$ and $R_4$, which are identical or different, each represents a straight-chained or branched ($C_6$-$C_{19}$)alkyl radical or straight-chained or branched ($C_6$-$C_{19}$)-alkenyl radical,

their enantiomers, diastereoisomers and epimers, and also their addition salts with a pharmaceutically acceptable acid.

**2.** Compound of formula (I) according to claim 1, wherein $R_2$ represents the group

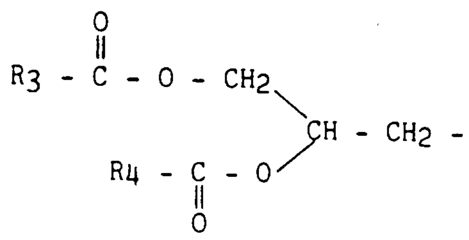

,

its enantiomers and diastereoisomers, and also its addition salts with a pharmaceutically acceptable acid.

**3.** Compound of formula (I) according to claim 1, wherein $R_3$-CO and $R_4$-CO, which are identical, each represents a stearoyl group, its enantiomers and diastereoisomers, and also its addition salts with a pharmaceutically acceptable acid.

**4.** Compound of formula (I) according to claim 1, wherein $R_3$-CO and $R_4$-CO, which are identical, each represents a myristoyl group, its enantiomers and diastereoisomers, and also its addition salts with a pharmaceutically acceptable acid.

**5.** Compound of formula (I) according to claim 1, wherein $R_3$-CO and $R_4$-CO, which are identical, each represents a palmitoyl group, its enantiomers and diastereoisomers, and also its addition salts with a pharmaceutically acceptable acid.

**6.** Compounds of formula (I) according to claim 1 wherein $R_1$ represents a hydrogen atom, their enantiomers, diastereoisomers and also their addition salts with a pharmaceutically acceptable acid.

**7.** Process for the preparation of compounds of formula (I) according to claim 1 wherein $R_2$ represents the group (A), characterised in that there is used as starting material p-[bis(2-chloroethyl)amino]phenylalanine of formula (II), in racemic form or in the form of the pure enantiomer, the amine function of which has been protected by a conventional protecting group :

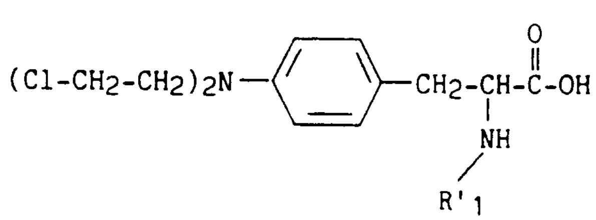

(II)

wherein $R'_1$ represents a straight-chained or branched ($C_1$-$C_6$)alkoxycarbonyl radical, or a phenylalkoxycarbonyl radical (unsubstituted or substituted on the phenyl nucleus by one or more halogen atoms or alkyl, alkoxy or trihaloalkyl groups),
which is reacted with the 1,3-dioxolane of formula (III) in racemic form or in the form of the pure enantiomer :

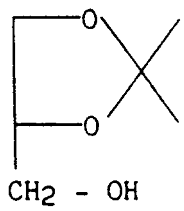

(III)

in the presence of a peptide synthesis coupling agent such as benzotriazol-1-yloxy-trisdimethylaminophosphonium hexafluorophosphate (BOP) and of diisopropylethylamine in dichloromethane,
to yield the 1,3-dioxolane of formula (IV) :

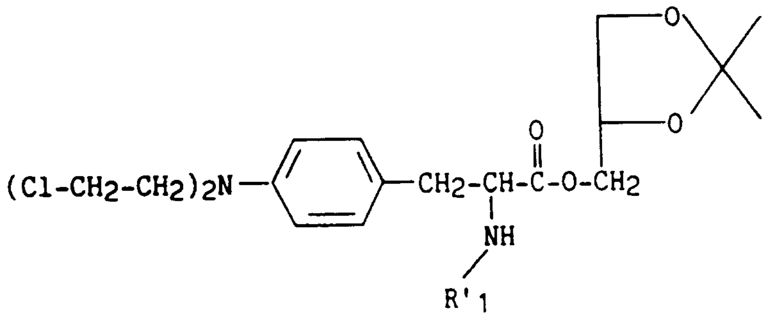

(IV)

wherein $R'_1$ is as defined above,
which is converted, in acid medium, into the corresponding diol of formula (V) :

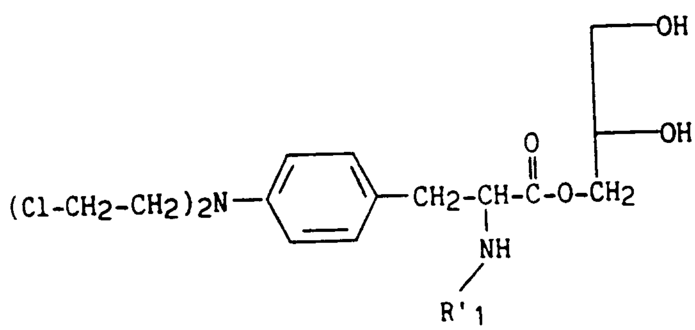

(V)

wherein $R'_1$ is as defined above,
which is reacted, according to the nature of the compounds of formula (I) it is desired to obtain :

- <u>either</u>, in the case where $R_3$ and $R_4$ are identical : with 2 equivalents of the acid chloride of formula (VI)

  $R_3$-CO-Cl (VI),

  wherein $R_3$ is as defined for formula (I), in the presence of a base,
  to yield the compound of formula (I/a), a particular case of compounds of formula (I),

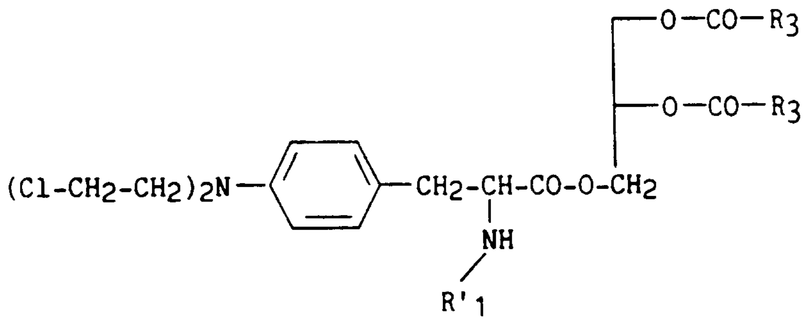

(I/a)

wherein $R'_1$ and $R_3$ are as defined above,

- or, in the case where $R_3$ and $R_4$ are different: with one equivalent of the acid chloride of formula (VI) described above,
to yield, after separation of the mono- and diacylated compounds, the compound of formula :

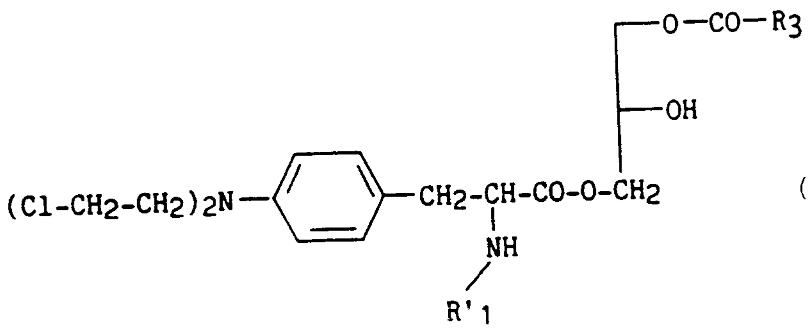

(VII)

wherein $R'_1$ and $R_3$ are as defined above,
which is reacted with one equivalent of the acid chloride of formula (VIII) :

$$R_4 - CO - Cl \qquad (VIII)$$

in the presence of a base to yield the compound of formula (I/b), a particular case of compounds of formula (I),

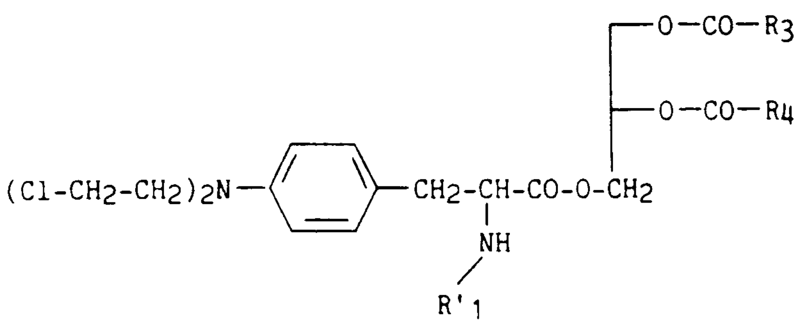

(I/b)

wherein $R'_1$, $R_3$ and $R_4$ are as defined above,

which compounds of formula (I/a) or (I/b) may, if desired, be subjected to acid hydrolysis or to hydrogen-olysis to yield, respectively, compounds of formula (I/c) or (I/d), a particular case of compounds of formula (I) :

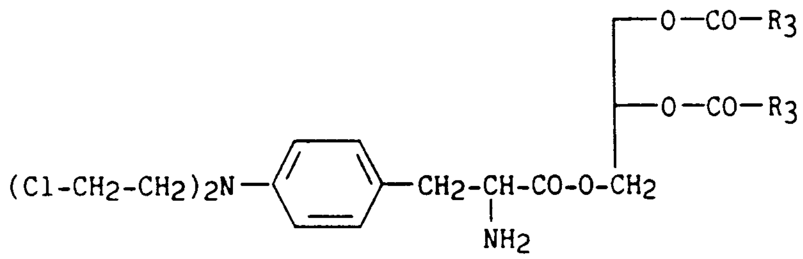

(I/c)

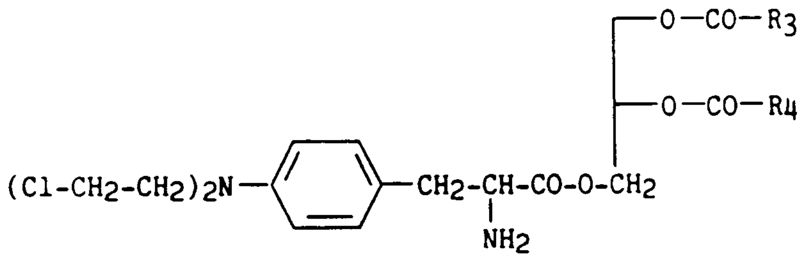

(I/d)

wherein $R_3$ and $R_4$ are as defined for formula (I),
which compounds of formula (I/a), (I/b), (I/c) or (I/d) :

- are purified, where necessary, according to a conventional method of purification,
- are separated, if desired, into their isomers according to a conventional method of purification,
- and are converted, if desired, into their addition salts with a pharmaceutically acceptable acid.

8. Process for the preparation of compounds of formula (I) according to claim 1 wherein $R_2$ represents the group (A), characterised in that there is used as starting material a compound of formula (IX) :

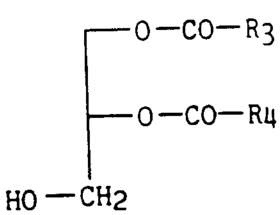

(IX)

wherein $R_3$ and $R_4$ are as defined for formula (I), which is reacted, in the presence of a peptide synthesis coupling agent such as benzotriazol-1-yloxy-trisdimethylaminophosphonium hexafluorophosphate (BOP) and of diisopropylethylamine in dichloromethane, with p-[bis(2-chloroethyl)amino]phenylalanine of formula (II), in racemic form or in the form of the pure enantiomer, the amine function of which has been protected by a conventional protecting group :

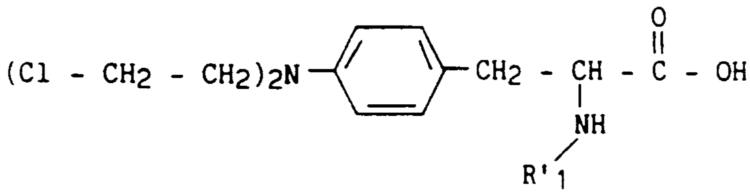

(II)

wherein $R'_1$ represents a straight-chained or branched $(C_1\text{-}C_6)$alkoxycarbonyl radical, or a phenylalkoxycarbonyl radical (unsubstituted or substituted on the phenyl nucleus by one or more halogen atoms or alkyl, alkoxy or trihaloalkyl groups),
to yield the compound of formula (I'), a particular case of compounds of formula (I) :

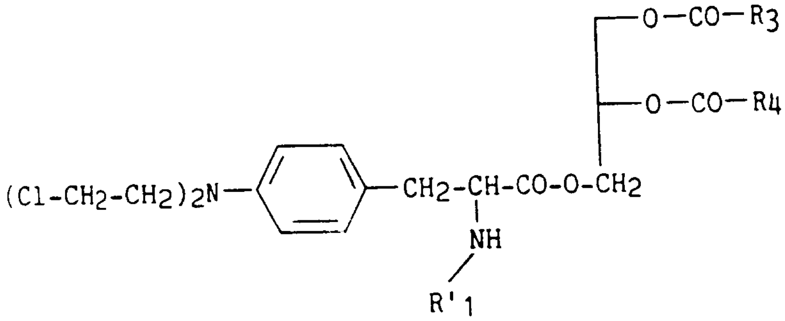

(I')

wherein $R'_1$, $R_3$, $R_4$ are as defined above, which may, if desired, be subjected to acid hydrolysis, or to hydrogenolysis, to yield, respectively, the compounds of formula (I"), a particular case of compounds of formula (I) :

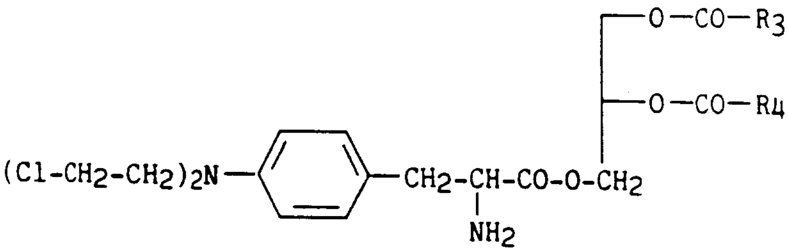

(I")

wherein $R_3$ and $R_4$ are as defined for formula (I),
which compounds of formula (I') or (I") :

- are purified, where necessary, according to a conventional method of purification,
- are separated, if desired, into their isomers according to a conventional method of purification,
- and are converted, if desired, into their addition salts with a pharmaceutically acceptable acid.

**9.** Process for the preparation of compounds of formula (I) according to claim 1 wherein $R_2$ represents the group (B) or (C), characterised in that there is used as starting material p-[bis(2-chloroethyl)amino]phenylalanine of formula (II), in racemic form or in the form of the pure enantiomer, the amine function of which has been protected by a conventional protecting group :

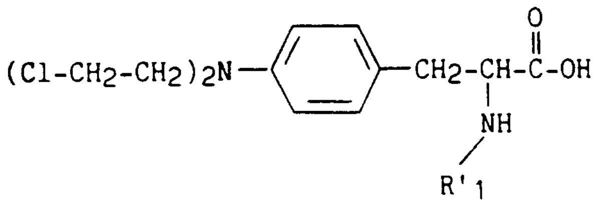

(II)

wherein R'$_1$ represents a straight-chained or branched ($C_1$-$C_6$)alkoxycarbonyl radical, or a phenylalkoxycarbonyl radical (unsubstituted or substituted on the phenyl nucleus by one or more halogen atoms or alkyl, alkoxy or trihaloalkyl groups),
which is reacted in the presence of isoprenyl chloroformate, triethylamine and 4-dimethylaminopyridine :

- either with the compound of formula (Xa) :

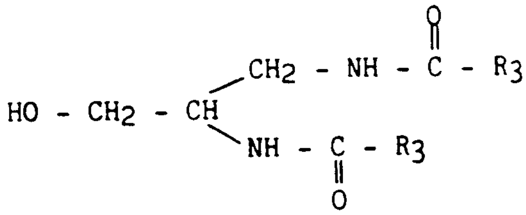

(Xa)

- or with the compound of formula (Xb) :

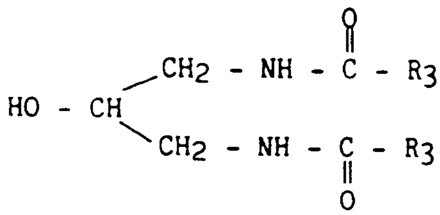

(Xb)

to yield, respectively, compounds of formula (I/e) or (I/f), a particular case of compounds of formula (I) :

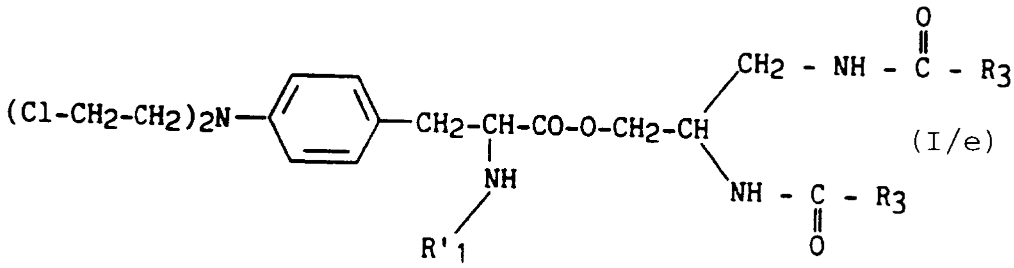

(I/e)

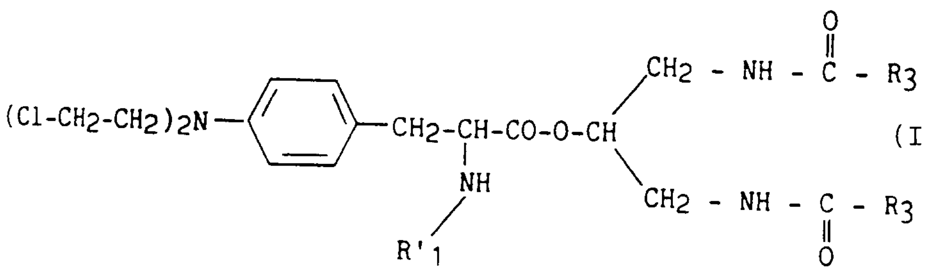

(I/f)

which compounds of formula (I/e) or (I/f) may, if desired, be subjected to acid hydrolysis or to hydrogen-olysis to yield, respectively, the compounds of formula (I/g) or (I/h), a particular case of compounds of formula (I) :

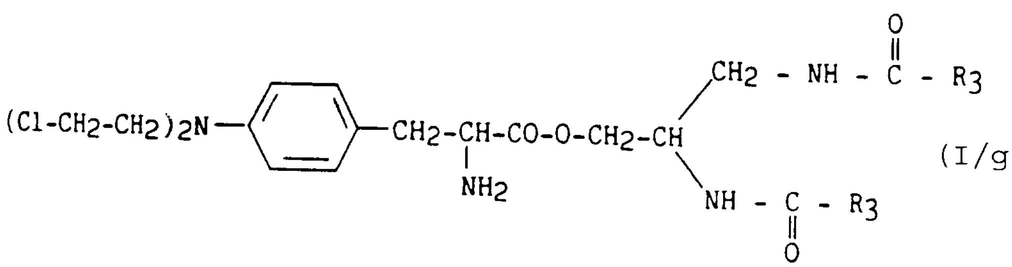

(I/g)

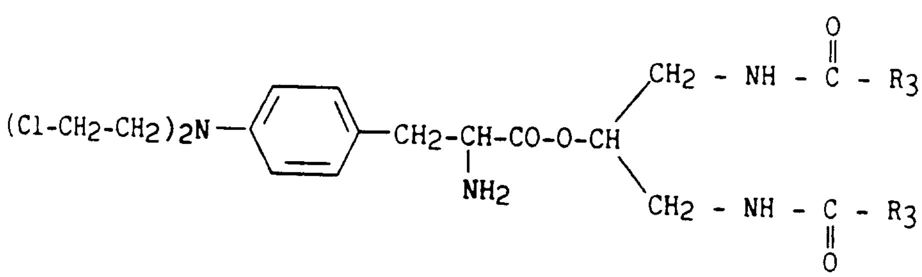

(I/h),

which compounds of formula (I/e), (I/f), (I/g) or (I/h) :

- are purified, where necessary, according to a conventional method of purification,
- are separated, if desired, into their isomers according to a conventional method of purification,
- and are converted, if desired, into their addition salts with a pharmaceutically acceptable acid.

**10.** Pharmaceutical compositions comprising as active ingredient at least one compound according to any one of claims 1 to 6, alone or in combination with one or more inert, non-toxic, pharmaceutically acceptable carriers.

**11.** Pharmaceutical compositions according to claim 10 for use in the treatment of cancer.

**Patentansprüche**

**1.** Verbindungen der Formel (I):

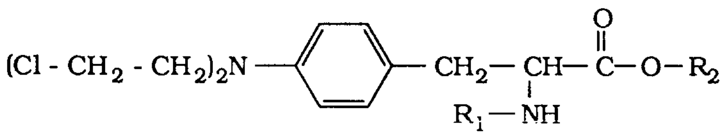

(I)

in der:

$R_1$ ein Wasserstoffatom, eine geradkettige oder verzweigte ($C_1$-$C_6$)-Alkoxycarbonylgruppe oder eine Phenylalkoxycarbonylgruppe (die gegebenenfalls am Phenylkern durch eines oder mehrere Halogenatome oder Alkyl-, Alkoxy- oder Trihalogenalkyl-gruppen substituiert ist),

$R_2$ eine der folgenden Gruppen:

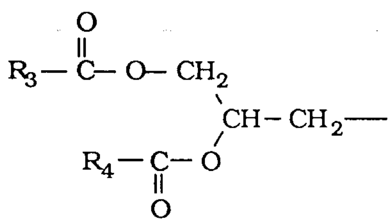

(A)

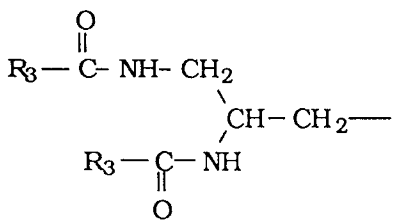

(B)

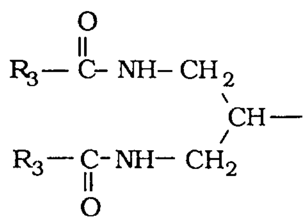

(C)

in denen:

$R_3$ oder $R_4$, die gleichartig oder verschieden sein können, eine geradkettige oder verzweigte ($C_6$-$C_{19}$)-Alkylgruppe oder eine geradkettige oder verzweigte ($C_6$-$C_{19}$)-Alkenylgruppe bedeuten,

deren Enantiomere, Diastereoisomere und Epimere sowie deren Additionssalze mit einer pharmazeutisch annehmbaren Säure.

2. Verbindung der Formel (I) nach Anspruch 1, worin $R_2$ eine Gruppe der Formel darstellt:

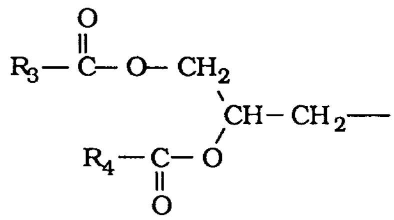

deren Enantiomere und Diastereoisomere sowie deren Additionssalze mit einer-pharmazeutisch annehmbaren Säure.

3. Verbindung der Formel (I) nach Anspruch 1, worin $R_3$-CO und $R_4$-CO, die gleichartig sind, jeweils eine Stearoylgruppe bedeuten, deren Enantiomere und Diastereoisomere sowie deren Additionssalze mit einer pharmazeutisch annehmbaren Säure.

4. Verbindung der Formel (I) nach Anspruch 1, worin $R_3$-CO und $R_4$-CO, die identisch sind, jeweils eine Myristoylgruppe bedeuten, deren Enantiomere und Diastereoisomere sowie deren Additionssalze mit einer pharmazeutisch annehmbaren Säure.

5. Verbindung der Formel (I) nach Anspruch 1, worin $R_3$-CO und $R_4$-CO, die identisch sind, jeweils eine Palmitoylgruppe darstellen, deren Enantiomere und Diastereoisomere sowie deren Additionssalze mit einer pharmazeutisch annehmbaren Säure.

6. Verbindung der Formel (I) nach Anspruch 1, worin $R_1$ ein Wasserstoffatom darstellt, deren Enantiomere und Diastereoisomere sowie deren Additionssalze mit einer pharmazeutisch annehmbaren Säure.

7. Verfahren zur Herstellung der Verbindungen der Formel (I) nach Anspruch 1, worin $R_2$ die Gruppe (A) darstellt, **dadurch gekennzeichnet**, daß man als Ausgangsprodukt p-[Bis(2-(chlorethyl)-amino]-phenylalanin der Formel (II) in racemischer oder reiner enantiomerer Form verwendet, dessen Aminogruppe man durch eine klassische Schutzgruppe geschützt hat:

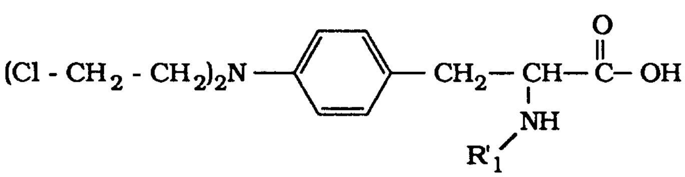

(II)

in der $R'_1$ eine geradkettige oder verzweigte ($C_1$-$C_6$)-Alkoxycarbonylgruppe oder eine Phenylalkoxylcarbonylgruppe (die gegebenenfalls am Phenylkern durch eines oder mehrere Halogenatome oder Alkyl-, Alkoxy- oder Trihalogenalkyl-gruppen substituiert ist) bedeutet,
welches man mit dem 1,3-Dioxolan der Formel (III):

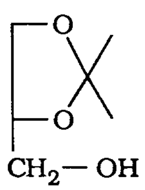

(III)

in racemischer Form oder in reiner enantiomerer Form in Gegenwart eines Kupplungsmittels für die Peptidsynthese, wie Benztriazol-1-yl-oxy-trisdimethylaminophosphoniumhexafluorphosphat (BOP) und Diisopropylethylamin in Dichlormethan umsetzt

zur Bildung des 1,3-Dioxolans der Formel (IV):

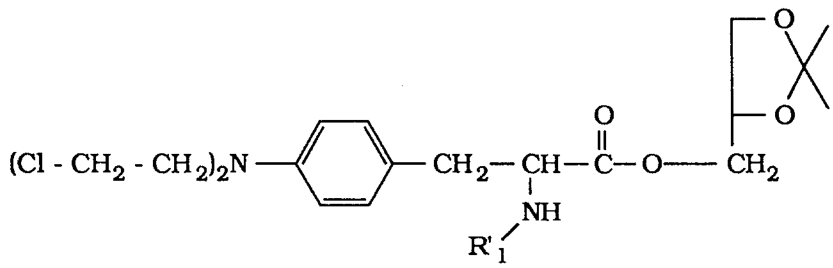

(IV)

in der $R'_1$ die oben angegebenen Bedeutungen besitzt, welches man in saurem Medium in das entsprechende Diol der Formel (V) umwandelt:

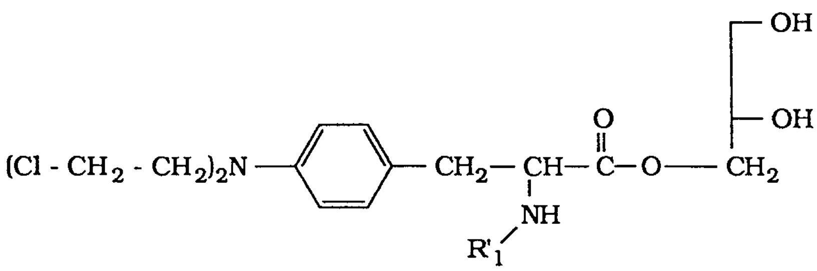

(V)

in der $R'_1$ die oben angegebenen Bedeutungen besitzt, welches man in Abhängigkeit von der Art der herzustellenden Verbindungen der Formel (I) mit:

- <u>entweder</u>, wenn $R_3$ und $R_4$ identisch sind:
  2 Äquivalenten des Säurechlorids der Formel (VI):

  $$R_3 - CO - Cl \qquad (VI)$$

  in der $R_3$ die bezüglich der Formel (I) angegebenen Bedeutungen besitzt, in Gegenwart einer Base zu der Verbindung der Formel (I/a) umsetzt, einem Sonderfall der Verbindungen der Formel (I):

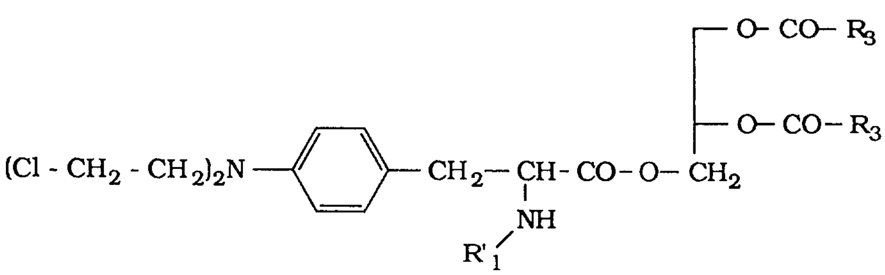

(I/a)

  in der $R'_1$ und $R_3$ die oben angegebenen Bedeutungen besitzen,
- <u>oder</u>, wenn $R_3$ und $R_4$ verschiedenartig sind:
  mit einem Äquivalent des oben beschriebenen Säurechlorids der Formel (VI) umsetzt, so daß man nach der Trennung der mono- und diacylierten Verbindungen die Verbindung der Formel:

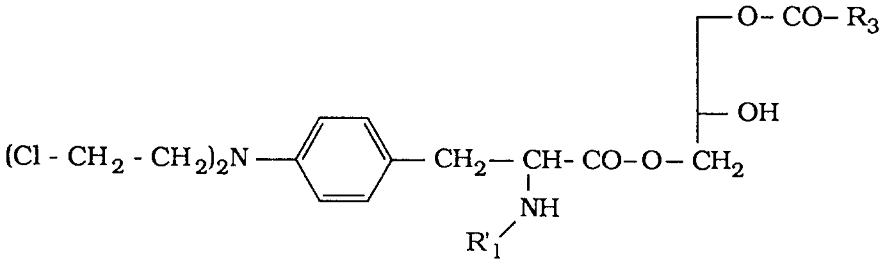

(VII)

erhält, in der $R'_1$ und $R_3$ die oben angegebenen Bedeutungen besitzen, welche man mit einem Äquivalent des Säurechlorids der Formel (VIII):

$R_4$ - CO - Cl (VIII)

in Gegenwart einer Base zu der Verbindung der Formel (I/b) umsetzt, einem Sonderfall der Verbindungen der Formel (I):

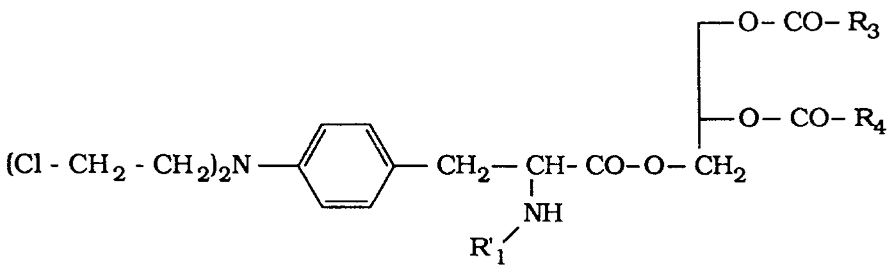

(I/b)

in der $R'_1$, $R_3$ und $R_4$ die oben angegebenen Bedeutungen besitzen,
welche Verbindungen der Formeln (I/a) oder (I/b) gewünschtenfalls einer sauren Hydrolyse oder einer Hydrogenolyse unterworfen werden können, so daß man zu den Verbindungen der Formel (I/c) bzw. (I/d) gelangt, einem Sonderfall der Verbindungen der Formel (I):

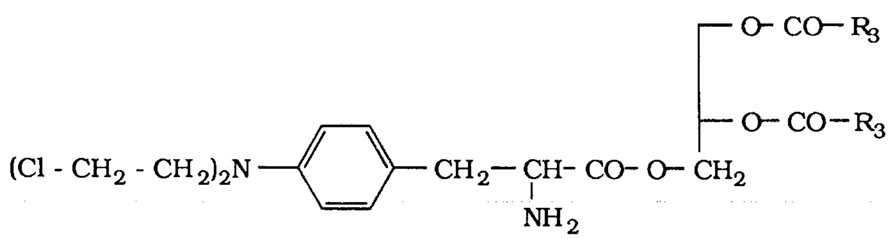

(I/c)

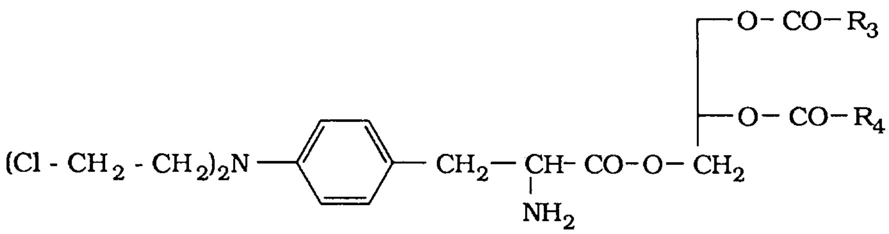

(I/d)

worin $R_3$ und $R_4$ die bezüglich der Formel (I) angegebenen Bedeutungen besitzen,

welche Verbindungen der Formeln (I/a), (I/b), (I/c) oder (I/d):

- man gegebenenfalls mit Hilfe einer klassischen Reinigungsmethode reinigt,

- gewünschtenfalls mit Hilfe einer klassischen Reinigungsmethode in die Isomeren auftrennt und
- gegebenenfalls in ihre Additionssalze mit einer pharmazeutisch annehmbaren Säure umwandelt.

**8.** Verfahren zur Herstellung der Verbindungen der Formel (I) nach Anspruch 1, worin $R_2$ die Gruppe (A) darstellt, **dadurch gekennzeichnet**, daß man als Ausgangsprodukt eine Verbindung der Formel (IX) einsetzt:

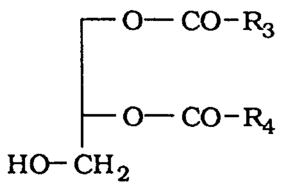

(IX)

in der $R_3$ und $R_4$ die bezüglich der Formel (I) angegebenen Bedeutungen besitzen, welche man in Gegenwart eines Kupplungsmittels für die Peptidsynthese, wie Benzotriazol-1-yl-oxy-trisdimethylaminophosphoniumhexafluorohosphat (BOP) und Diisopropylethylamin in Dichlormethan mit p-[Bis(2-chlorethyl)-amino]-phenylalanin der Formel (II) in racemischer oder reiner enantiomerer Form, deren Aminogruppe man durch eine klassische Schutzgruppe geschützt hat:

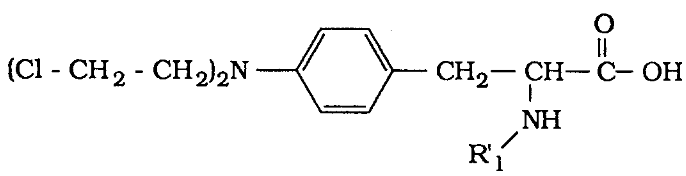

(II)

in der $R'_1$ eine geradkettige oder verzweigte ($C_1$-$C_6$)-Alkoxygruppe oder eine Phenylalkoxycarbonylgruppe (die gegebenenfalls am Phenylkern durch eines oder mehrere Halogenatome oder Alkyl-, Alkoxy- oder Trihalogenalkylgruppen substituiert ist)
zu der Verbindung der Formel (I') umsetzt, einem Sonderfall der Verbindungen der Formel (I):

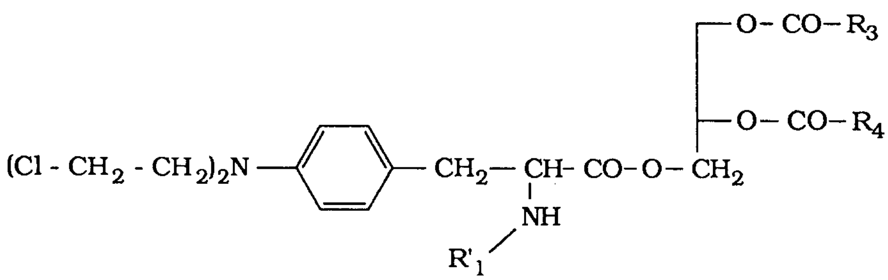

(I')

in der $R'_1$, $R_3$ und $R_4$ die oben angegebenen Bedeutungen besitzen, welche man gewünschtenfalls einer sauren Hydrolyse oder einer Hydrogenolyse unterwirft, so daß man die Verbindungen der Formel (I'') erhält, einem Sonderfall der Verbindungen der Formel (I):

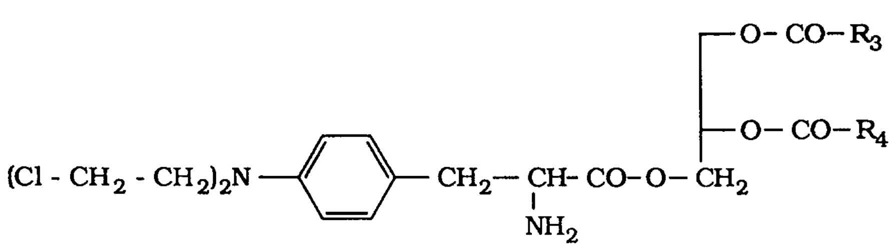

(I'')

in der $R_3$ und $R_4$ die bezüglich der Formel (I) angegebenen Bedeutungen besitzen, welche Verbindungen der Formeln (I') oder (I") man:

- gegebenenfalls mit Hilfe einer klassischen Reinigungsmethode reinigt,
- gegebenenfalls mit Hilfe einer klassischen Reinigungsmethode in die Isomeren auftrennt und
- gegebenenfalls in ihre Säureadditionssalze mit einer pharmazeutisch annehmbaren Säure umwandelt.

**9.** Verfahren zur Herstellung der Verbindungen der Formel (I) nach Anspruch 1, worin $R_2$ die Gruppe (B) oder (C) darstellt, **dadurch gekennzeichnet**, daß man als Ausgangsprodukt p-[Bis(2-chlorethyl)-amino]-phenylalanin der Formel (II) in racemischer oder reiner enantiomerer Form verwendet, dessen Aminogruppe man durch eine klassische Schutzgruppe geschützt hat:

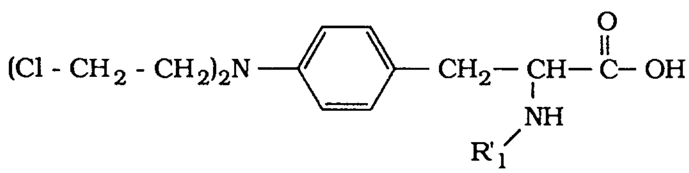

(II)

in der $R'_1$ eine geradkettige oder verzweigte ($C_1$-$C_6$)-Alkoxycarbonylgruppe oder eine Phenylalkoxycarbonylgruppe (die gegebenenfalls am Phenylkern durch eines oder mehrere Halogenatome oder Alkyl-, Alkoxy- oder Trihalogenalkylgruppen substituiert ist) bedeutet,
welches man in Gegenwart von Chlorameisensäureisoprenylester, Triethylamin und 4-Dimethylaminopryridin:

- <u>entweder</u> mit der Verbindung der Formel (Xa):

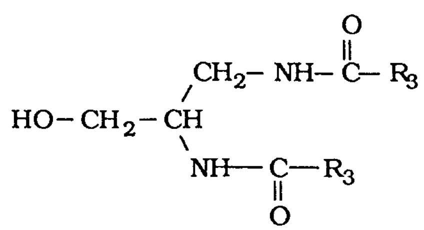

(Xa)

- <u>oder</u> mit der Verbindung der Formel (Xb):

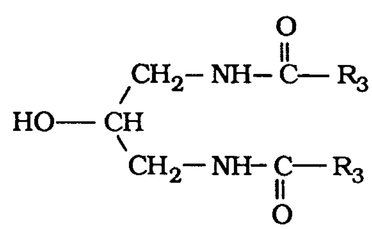

(Xb)

umsetzt, so daß man die Verbindungen der Formel (I/e) bzw. (I/f) erhält, einem Sonderfall der Verbindungen der Formel (I):

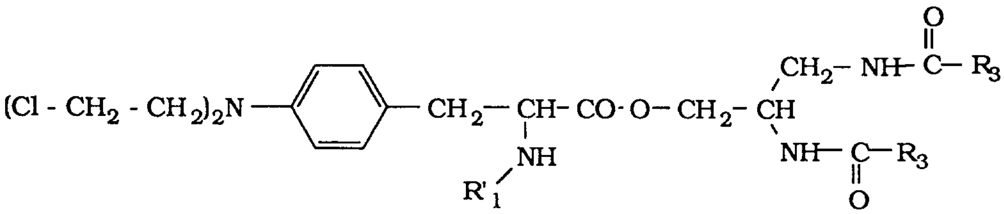

(I/e)

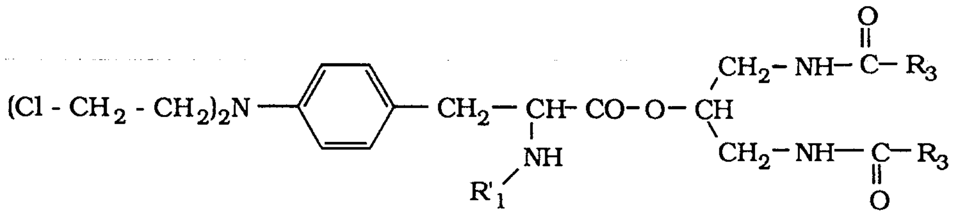

(I/f)

welche Verbindungen der Formeln (I/e) oder (I/f) man gewünschtenfalls einer sauren Hydrolyse oder einer Hydrogenolyse unterwerfen kann, so daß man die Verbindungen der Formeln (I/g) bzw. (I/h) erhält, einem Sonderfall der Verbindungen der Formel (I):

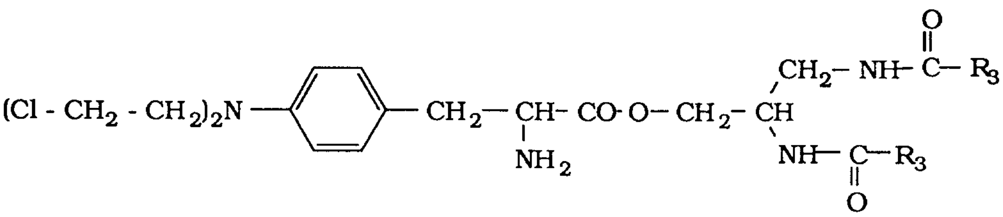

(I/g)

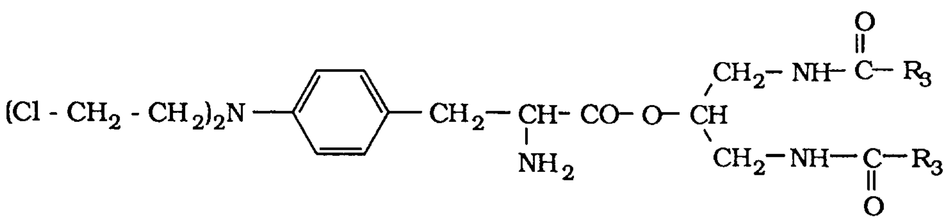

(I/h)

welche Verbindungen der Formeln (I/e), (I/f), (I/g) oder (I/h) man:

- gewünschtenfalls mit Hilfe einer klassischen Reinigungsmethode reinigt,
- gewünschtenfalls mit Hilfe einer klassischen Reinigungsmethode in die Isomeren auftrennt und
- gegebenenfalls in ihre Additionssalze mit einer pharmazeutisch annehmbaren Säure umwandelt.

**10.** Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien.

**11.** Pharmazeutische Zubereitungen nach Anspruch 1 zur Behandlung von Krebs.